# EUROPEAN PATENT SPECIFICATION

(11) **EP 3 259 598 B1**
(45) Date of publication and mention of the grant of the patent: **29.07.2020**
(21) Application number: 16752004.8
(22) Date of filing: 15.02.2016
(51) Int. Cl.: G01N 33/574

(54) **MULTIFUNCTIONAL MAGNETO-POLYMERIC NANOSYSTEMS FOR RAPID TARGETING, ISOLATION, DETECTION AND SIMULTANEOUS IMAGING OF CIRCULATING TUMOR CELLS**
MULTIFUNKTIONELLE MAGNETOPOLYMERE NANOSYSTEME FÜR SCHNELLES TARGETING, ISOLIERUNG, NACHWEIS UND GLEICHZEITIGE BILDGEBUNG VON ZIRKULIERENDEN TUMORZELLEN
NANOSYSTÈMES MAGNÉTO-POLYMÈRES MULTIFONCTIONNELS POUR LE CIBLAGE RAPIDE, L'ISOLEMENT, LA DÉTECTION ET L'IMAGERIE SIMULTANÉE DE CELLULES TUMORALES CIRCULANTES

(30) Priority: 19.02.2015 IN 538MU2015
(43) Date of publication of application: 27.12.2017
(73) Proprietor: Actorius Innovations and Research Pvt. Ltd., Mumbai 400076 (IN)
(72) Inventor: KHANDARE, Jayant Jagannath, Mumbai 400064 (IN); BANERJEE, Shashwat, Kalyan 421306 (IN); PADIGARU, Muralidhara, Bangalore 560064 (IN); KHUTALE, Ganesh, Pune 410502 (IN)
(74) Representative: Kasche, André
(86) International application number: PCT/IB2016/050779
(87) International publication number: WO 2016/132265

(56) References cited:
- WO-A2-2008/073856
- US-A1- 2012 094 275
- US-A1- 2014 087 936
- BALASUBRAMANIAM ET AL.: 'Poly(N-isopropylacrylamide)-Coated Superparamagnetic Iron Oxide Nanoparticles: Relaxometric and Fluorescence Behavior Correlate to Temperature-Dependent Aggregation' CHEM. MATER. vol. 23, no. 14, 2011, pages 3348 - 3356, XP055362195
- XU ET AL.: 'Dendrimer Advances for the Central Nervous System Delivery of Therapeutics' ACS CHEM NEUROSCI vol. 5, no. 1, 2014, pages 2 - 13, XP055439825
- MODUGNO ET AL.: 'Carbon nanomaterials combined with metal nanoparticles for theranostic applications' BRITISH JOURNAL OF PHARMACOLOGY vol. 172, no. 4, 2015, pages 975 - 991, XP055439827
- KHODADUST ET AL.: 'PAMAM dendrimer-coated iron oxide nanoparticles: synthesis and characterization of different generations' J NANOPART RES vol. 15, no. 1488, 2013, pages 1 - 13, XP055439830
- XU ET AL.: 'Water-Soluble Iron Oxide Nanoparticles with High Stability and Selective Surface Functionality' LANGMUIR vol. 27, 01 January 2011, pages 8990 - 8997, XP055490907 DOI: 10.1021/LA201652H
- ISSADORE ET AL.: 'Ultrasensitive clinical enumeration of rare cells ex vivo using a mirco-Hall detector' SCI TRANSL MED. vol. 4, no. 141, 2012, page 141RA92, XP055357516
- BHANA ET AL.: 'Nanotechnology for enrichment and detection of circulating tumor cells' NANOMEDICINE (LOND vol. 10, no. 12, 2015, pages 1973 - 1990, XP055439848

## Description

### Field of Invention

This application is related to a biofunctional multicomponent nanosystem for specific targeting, rapid isolation and simultaneous high resolution imaging of cancer cells.

### Background

Counting of metastatic cells is of key importance in predicting patient prognosis, monitoring and assessing therapeutic outcomes (Cristofanilli et al. N. Engl. J. Med. 2004, 351, 781).

However, presence of metastatic cancer cells in blood stream is extremely rare making their isolation and detection very challenging. These metastatic cells referred to as Circulating Tumor Cells (CTC) are known to be associated with short survival in hematological cells and have been a subject of research especially for developing rapid and cost-effective diagnostics in cancer biology. CTC-based diagnosis is very valuable as it provides insight into tumor, critical for designing therapeutic intervention.

Technical advances have allowed detection of CTC to a certain extent. Currently, the immunomagnetic separation of CTC (CellSearch assay) is FDA approved. However, more detection techniques are explored due to the need to detect different forms of cancer cells, reduce cost, and increase efficiency. These include flow cytometry (Allan et al Cytom Part A, 2005, 65:4), size-based filtration systems (Jacob et al, Biotechnology and Bioengineering, 2009, 102: 521) and microfluidic devices (J Chromatogr A, 2007, 1162: 154). But these techniques are not efficient in rapid isolation and characterization of CTCs. Wang et al have demonstrated a CTC assay capable of enumerating CTC in whole-blood samples from prostate cancer patients wherein cell-affinity substrates with capture agent-coated silicon nanowire substrates have been used to immobilize CTCs (Adv Materials, 2011, 23: 4788-92). Further, nanovelcro chip capturing non-small cell lung cancer (NSCLC) CTCs from blood and recovering the nanosubstrate immobilized NSCLC CTCs upon treatment of nuclease solution is also described (Shen et al, Advanced Materials, 2013, 25: 2368-73). The present disclosure

The provides a Magneto Polymeric- Nanosystem (MPNS) consisting of carbon allotropes including carbon nanotube and or graphene which reliably captures cancer cells mediated by specific antibody/ies and specific targeting components from the blood samples with greater interactions with cancer cells which is hitherto not known in any other detection system. For example, Banerjee et al provide a multicomponent magneto-dendritic nanosystem (MDNS) for rapid tumor cell targeting, isolation and high resolution imaging (Advanced Healthcare materials, 2013, 2(6): 800). But this kind of system lacks ideal traits, including carbon nanotube (CNT) as a platform and an additional polymer system such as poly(N isopropyl acrylamide (PNIPAM) and hyper branched polymers [(e.g. poly (amidoamine (PAMAM) dendrimers and polyglycerols), poly (ethylene glycols)] supporting the higher aqueous dispersibility of the multicomponentsand specific antibodies (eg.anti-Epithelium Cell Adhesion Molecules (EpCAM) which finally enhances the interactions with cancer

WO 2008/073856 A2 discloses systems, methods and compositions for targeted delivery of nanoparticles and/or agents to tissues, cells, and/or subcellular locales.

### Brief Description of Drawings

**Figure 1****.** (A) A typical TEM image of Fe₃O₄ nanoparticles. (B) Size distribution of the Fe₃O₄ nanoparticles was estimated from TEM images.
**Figure 2****.** ATR-IR spectra of (a) Fe₃O₄, (b) AIR-001, (c) AIR-002, (d) CNT-COOH, (e) AIR-010, (f) AIR-011, and (g) AIR-012.
**Figure 3****.** Dispersibility of AIR-072 in aqueous media.
**Figure 4****.** Normalized fluorescence spectra (λₑₓ = 600 nm) of free Cy5 and AIR-007. The dotted red line show the fluorescence peaks for free Cγ5.
**Figure 5****.** (A-G) Image of the remaining cell suspension after magnetic capture of the HCT116 cells. HCT116 cells found to remain in solution is shown by red dotted circle. (H) Image of the magnetically isolated HCT116 cells from cell media after 3 min incubation.
**Figure 6****.** Plot showing cells captured by MPNS in percentage.
**Figure 7****.** (A-C) Image of the remaining cell suspension after magnetic capture of the HCT116 cells. HCT116 cell found to remain in solution is shown by red dotted circle; (D,E) Images of the magnetically isolated HCT116 cells by using MPNS with (E) and without (D) EpCaM antibody from cell media after 3 min incubation, (F) Control.
**Figure 8****.** Plot showing cells captured by MPNS with (AIR-060) and without (AIR-011) EpCam antibody in percentage.
**Figure 9****.** Plot showing HCT116 cells captured from spiked cell suspension by MPNS with (AIR-060) and without (AIR-039) EpCam antibody in percentage.
**Figure 10****.** Image of the isolated HCT116 cells from cell media by MPNS with EpCam after 3 min incubation.
**Figure 11****.** Plot showing HCT116 cells captured by MPNS with (AIR-072) and without (AIR-071) EpCam antibody in percentage from clinically relevant CTC-like suspensions prepared in 1 x 10⁵:1 (hPBMC:HCT116) ratios.
**Figure 12****.** Immunostaining of CTC captured cells from peripheral blood cells of colon, rectal, lung and breast cancer subjects. Paraformaldehyde fixed, DAPI (blue), CK18 FITC (green) and DAPI+CK18 FITC positive (green & blue merge) of patient using CNT/graphene nanosystem based AIR methods.

### Details of the Invention

As a part of the design, three bio-functionalized nanosystems for specific targeting, rapid isolation and high-resolution imaging of cancer cells have been developed. The nanosystems are designed using 7 functional elements as provided below:
(i) transferrin (Tf)/EpCAM antibody or any other CTC specific or non-specific antibody targeting cancer cells and other biomolecules including protein, carbohydrate or small biologically relevant molecules,
(ii) iron oxide (Fe₃O₄) nanoparticles to allow magnetic isolation,
(iii) cyanine 5 NHS (Cy5) dye to enable high-resolution imaging of the isolated CTCs,
(iv) Poly(N isopropyl acrylamide) (PNIPAM)), a thermoresponsive polymer (exhibiting a lower critical solution temperature (LCST)) capable of affecting the conformational structural changes resulting in assisting cancer cell capture,to increase the dispersibility of the nanosystem,
(v) Carbon allotropes, exemplified by single/mutiwalled carbon nanotube (CNT) or nanohorns or Graphene or any other carbon allotropes for better interaction with cancer cells,
(vi)fourth generation (G4) hyperbranched polymers like dendrimers (poly(aminoamidine) (PAMAM) with 64 reactive sites (generation ∼ G4) and hyperbranched polymers (e.g. polyglycerols, polyiminesetc) to facilitate the simultaneous conjugation of multiple functional entities, and
(viii) glutathione (GSH) as a multifunctional reactive linker. We followed a multi-step process (Scheme 1, 2, 3 and 4) to synthesize the Magneto-Polymeric NanoSystems (MPNS) platform. By 'any other CTC specific antibody', it is meant any antibody in published literature that target cancer cells or novel antibody that may find a use in the future.

The present invention provides a biofunctional multicomponent nanosystem, a process of synthesizing same, diagnostic uses of the nanosystem and a kit comprising the nanosystem according to the appended claims.

### Synthesis of Fe₃O₄

Fe₃O₄ magnetic nanoparticles (MNP) were prepared by co-precipitating Fe²⁺ and Fe³⁺ ions by ammonia solution and treating under hydrothermal conditions.

### Anchoring of Glutathione (GSH) with Fe₃O₄ (AIR-001)

Fe₃O₄ dispersed in ultrapure water and methanol by sonication was mixed with GSH dissolved in ultrapure water. The mixture was then re-sonicated for 2 h. Fe₃O₄-GSH was then isolated by magnetic separation, washed with repeated cycles of excess de-ionized water (D.I.) water, and dried under vacuum. The conjugate will be denoted as AIR-001 in the following studies.

### Synthesis of Fe₃O₄-GSH-PAMAM G4 dendrimer conjugate (AIR-002)

AIR-001 was conjugated with PAMAM G4 dendrimer by (*N*-(3-dimethylaminopropyl)-*N-*ethyl carbodiimide hydrochloric acid) (EDCHCl) coupling method. PAMAM (G4) dendrimers are coupled with COOH, NH₂, OH or other reactive groups. The conjugate was then isolated by magnetic separation, washed with repeated cycles of D.I. water, and dried under vacuum. The conjugate is denoted as AIR-002 in the following studies.

### Synthesis of Fe₃O₄-GSH-PAMAM G4-CNT/mutiwalled carbon nanotube (CNT) or nanohorns or graphene or any other carbon allotropes conjugate (AIR-010)

AIR-002 was conjugated to CNT or graphene or nanohorns by EDC coupling method. The conjugate was then isolated by magnetic separation, washed with repeated cycles of D.I. water, and dried under vacuum. The conjugate is denoted as AIR-010 in the following studies.

### Synthesis of Fe₃O₄-GSH-PAMAM G4-CNT-or nanohorns or graphene-PNIPAM conjugate (AIR-054) (Scheme 2 and 4)

AIR-010 was conjugated to PNIPAM-COOH/NH₂/SH by EDC coupling method. The conjugate was then isolated by magnetic separation, washed with repeated cycles of D.I. water, and dried under vacuum. The conjugate is denoted as AIR-054 in the following studies.

### Synthesis of Fe₃O₄-GSH-PAMAM G4-CNTmutiwalled carbon nanotube (CNT) or nanohorns or graphene or any other carbon allotropes -Cy5 conjugate (AIR-011)

Cy5 NHS was conjugated with AIR-010 in presence of DIPEA at a pH of 7.8. The product was then isolated by magnetic separation, washed with repeated cycles of D.I. water and dried at room temperature under vacuum. The conjugate is denoted as AIR-011 in the following studies.

### Synthesis of Fe₃O₄-GSH-PAMAM G4-CNTmutiwalled carbon nanotube (CNT) or nanohorns or graphene or any other carbon allotropes - PNIPAM-Cy5 conjugate (AIR-055)

AIR-054 was conjugated to Cy5 NHS in presence of DIPEA at a pH of 7.8. The conjugate was then isolated by magnetic separation, washed with repeated cycles of D.I. water, and dried under vacuum. The conjugate is denoted as AIR-055 in the following studies.

### Synthesis of Fe₃O₄-GSH-PAMAM G4-CNT-mutiwalled carbon nanotube (CNT) or nanohorns or graphene or any other carbon allotropes Cy5-Tf conjugate (AIR-012)

AIR-011 was conjugated to transferrin (Tf) using EDC coupling method. The conjugate was then isolated by magnetic separation, washed with repeated cycles of D.I. water, and dried under vacuum. The final conjugate is denoted as AIR-012 in the following studies.

### Synthesis of Fe₃O₄-GSH-PAMAM G4-CNT-mutiwalled carbon nanotube (CNT) or nanohorns or graphene or any other carbon allotropes -Cy5-Tf conjugate (AIR-056)

AIR-055 was conjugated to transferrin (Tf) using EDC coupling method. The conjugate was then isolated by magnetic separation, washed with repeated cycles of D.I. water, and dried under vacuum. The final conjugate is denoted as AIR-056 in the following studies.

### Synthesis of Fe₃O₄-GSH-PAMAM G4-CNT-mutiwalled carbon nanotube (CNT) or nanohorns or Graphene or any other carbon allotropes Cy5-EpCam conjugate

AIR-011 was conjugated with EpCam antibody using EDC coupling method. The conjugate was then isolated by magnetic separation, washed with repeated cycles of D.I. water, and dried under vacuum. The final conjugate is denoted as AIR-060 in the following studies.

### Synthesis of Fe₃O₄-GSH-PAMAM G4-CNT-mutiwalled carbon nanotube (CNT) or nanohorns or Graphene or any other carbon allotropes -Cy5-EpCam conjugate

AIR-055 was conjugated with EpCam antibody using EDC coupling method. The conjugate was then isolated by magnetic separation, washed with repeated cycles of D.I. water, and dried under vacuum. The final conjugate is denoted as AIR-066 in the following studies.

### MPNS-cell interaction and imaging

HCT116 cells were plated at a density of 5x10² per 100 µl in 96 wells plate. HCT116 cells were treated with 500 µg of MPNS sufficiently diluted with suitable buffers and incubated on shaker for 3 minutes. Strong magnetic field was applied to separate MPNS and the supernatant cell media was transferred to another well in order to count the uncaptured cancer cells. The MPNS-captured and uncaptured cells were counted from the images of MPNS-captured and uncaptured cells using Leica Fluorescence Microscope to estimate the cancer cell capture efficiency of MPNS nanosystems.

### Estimation of capture efficiency from artificial CTC suspension

CTC samples were prepared by spiking HCT116 cells with human peripheral blood mononuclear cells (hPBMCs) at the ratio 1:1000 in 96 wells plate. Artificial CTC suspension was treated with 500 µg of MPNS (with and without EpCam) conjugate sufficiently diluted with suitable buffers and incubated on shaker for 3 minutes. Strong magnetic field was applied to separate MPNS and the supernatant cell media was transferred to another well in order to count the uncaptured cancer cells. The MPNS-captured and uncaptured cells were counted from the images of MPNS-captured and uncaptured cells using Fluorescence Microscope to estimate the cancer cell capture efficiency of MPNS nanosystems.

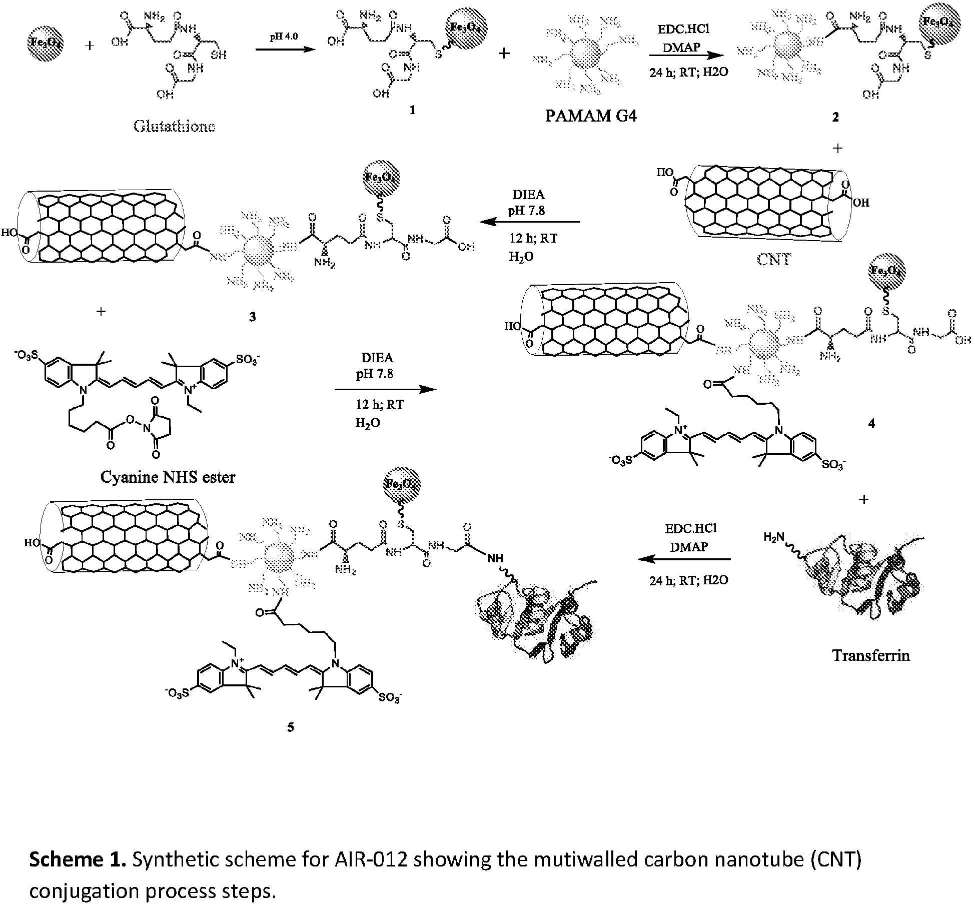

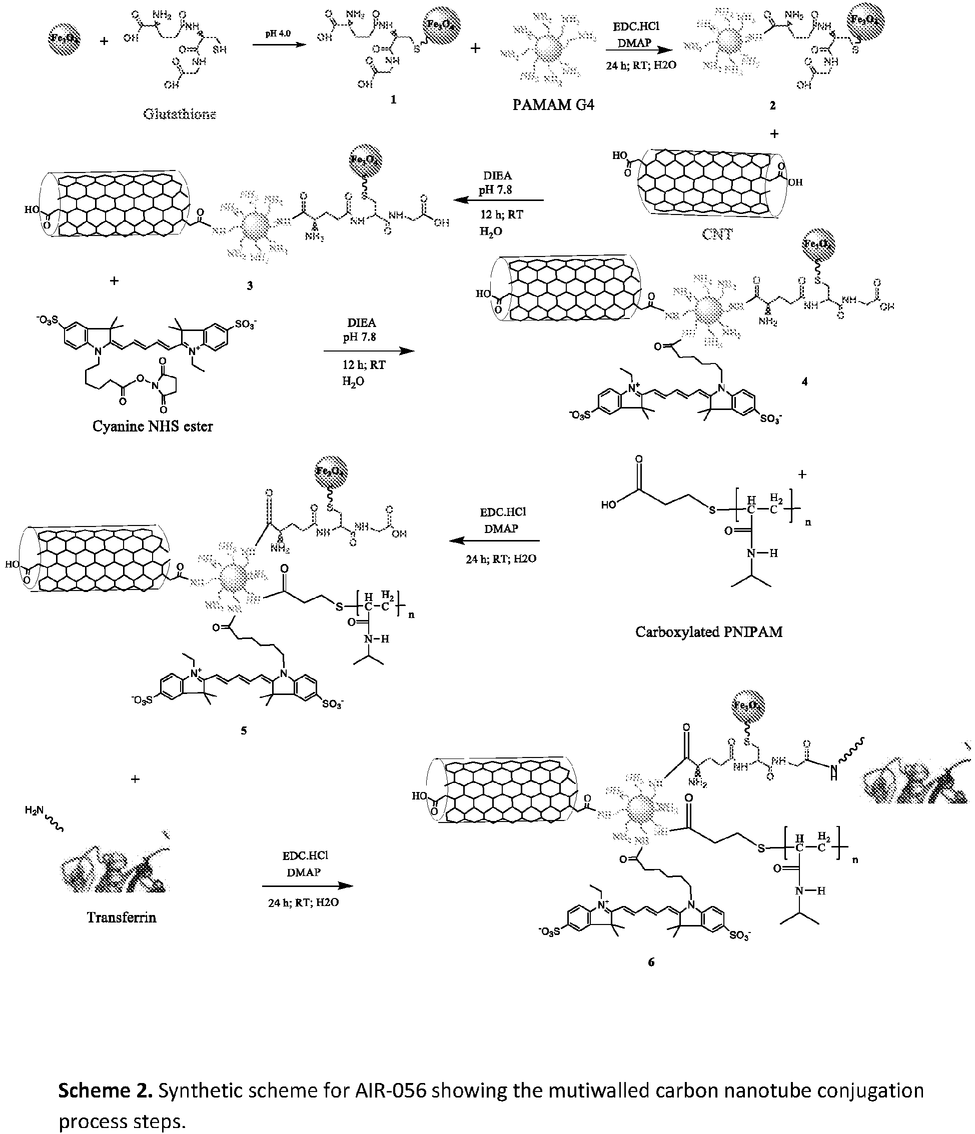

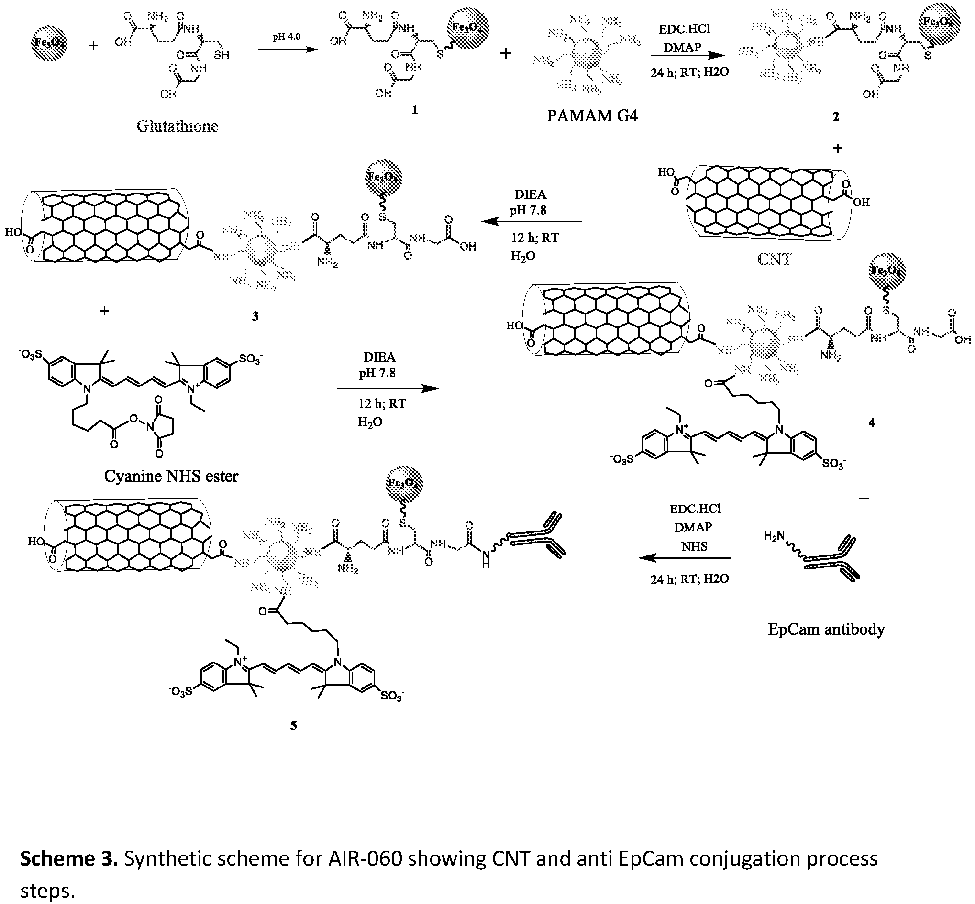

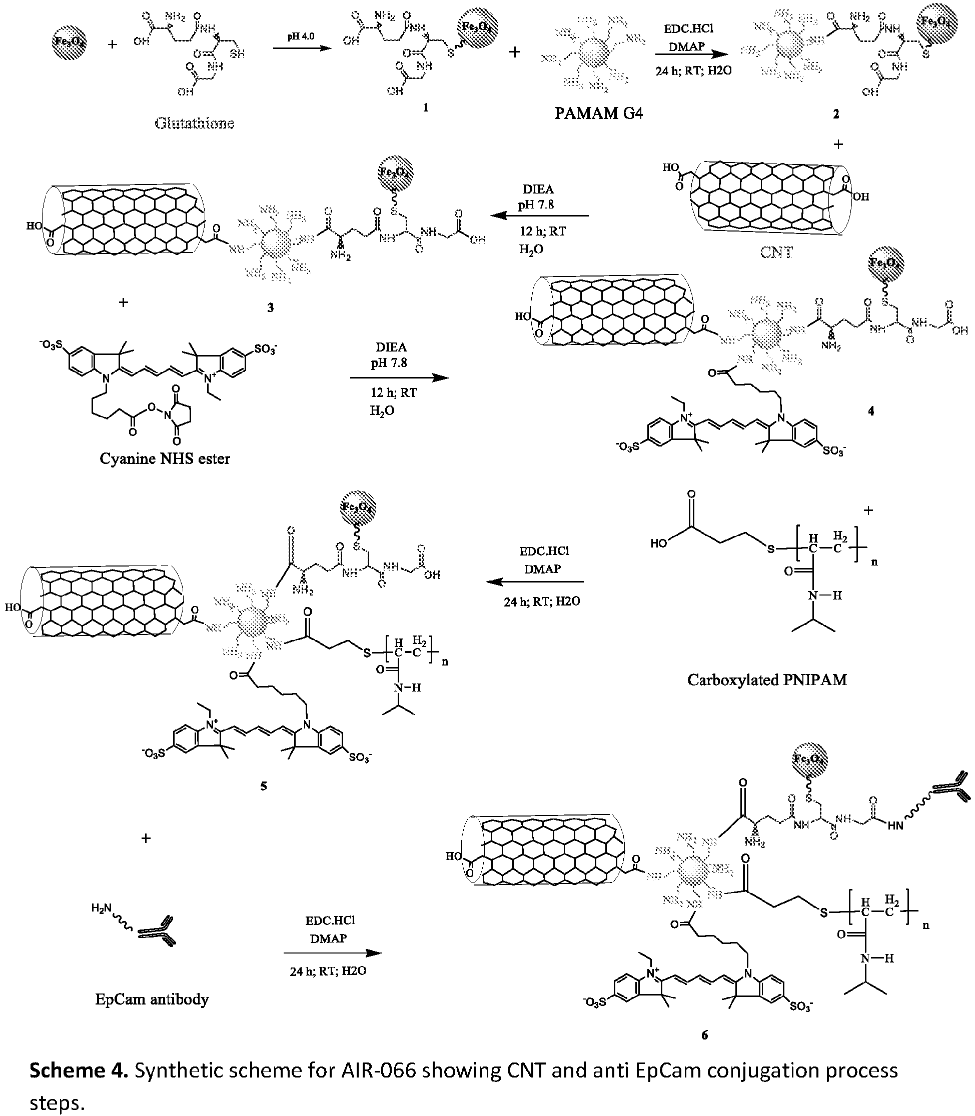

### Advantages of MPNS

The MPNS of the present invention demonstrate higher dispersibility in biologically relevant fluids and reliably capture cancer cells from CTC suspension of clinically relevant concentration with about 95% accuracy.
MPNS provides a convenient, cost-efficient and rapid capturing alternative of CTC for clinical samples.
Cell viability with MPNS platform is as high as 90% which is conducive to subsequently releasing the cells, culturing them, and performing molecular and clinical diagnosis.
Use of PNIPAM, a thermoresponsive smart polymer and PAMAM G4 dendrimer significantly enhances the dispersibility of the magnetic multicomponent system of the present invention. The multicomponent system imparts the conjugation of varied antibodies due to the chemical tunability. The system has simultaneous imaging probe through near infrared agent-Cyanine.

The overall impact of the MPNS cell capture technology is envisioned beyond the CTCs potential benefit in early diagnosis of diseases that are detected by few cell-capture technologies.
The multicomponent nano system provided here may also find applications in detecting other diseases by conjugating specific biomarkers and bioactive components. For example,
this technology platform can be extended to detection of other diseases specifically cardiovascular and infectious diseases by attaching specific antibodies to the polymeric nanosystem. More specifically, screening for Acute Myocardial Infarction by detecting Troponin T levels in blood using specific anti-troponin-magnetic systems or immunomagnetic separation of pathogenic organisms from environmental matrices.

### Definitions

Cells or antibodies as provided in this specification are any cells or antibodies that specifically target cancer cells. These can be biomolecule interacting antibodies that are already known, for example, published elsewhere, or novel antibodies or proteins.

Carbon allotropes as provided here include single or multiwalled carbon nanotubes (CNT), graphene or nanohorns. They will be in either oxidized or non-oxidized forms or functionalized with other reactive groups.

Fourth generation PAMAM (G4) dendrimers or polymers are polyglycerols, polyamines or reactive and modified hyperbranched polymers that are coupled to COOH, NH₂, OH or other reactive groups.

These dendrimers or hyperbranched polymers provide for simultaneous attachment of multiple functional groups.

Glutathione (GSH) as provided here serve as a multifunctional reactive linker. Other reactive linkers including citric acid, thiol functional small molecules, aliphatic reactive chains and other reactive amino acids can be used in the present invention.

### Examples:

### Characterization of MPNS

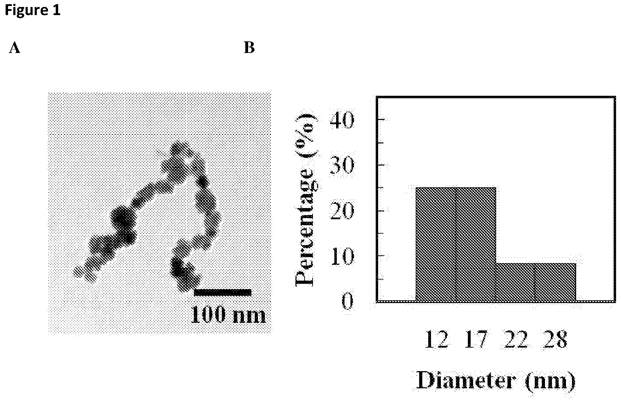

The structure of Fe₃O₄ nanoparticles was investigated by TEM as shown in **Figure 1****.** The average size of the Fe₃O₄ particles in the matrix is estimated to be ∼ 17 nm. The size distribution of the Fe₃O₄ nanoparticles is given in Figure 1.

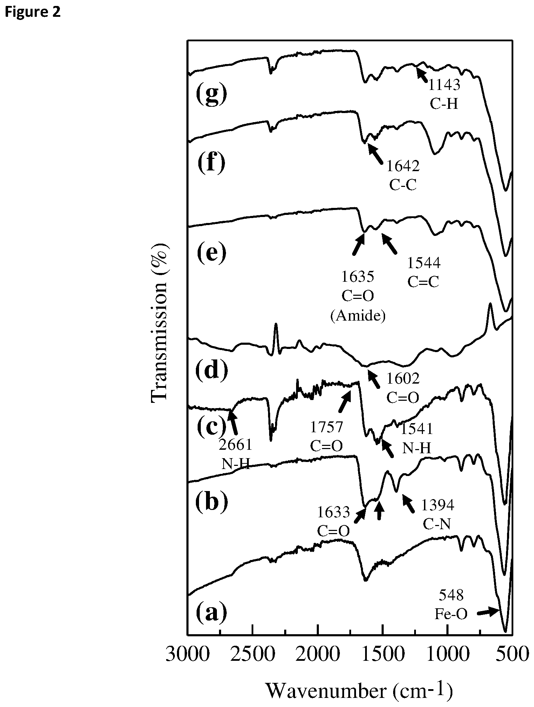

The surface chemistry of the nano conjugates was characterized by attenuated total reflectance (ATR-IR). As shown in **Figure 2** **(A,B)**, the spectrum of AIR-001, AIR-002, AIR-003, AIR-005, and AIR-007, AIR-012 showed new peaks compared to the preceding nano system due to the new component conjugation. Thus, the IR characterization proved successful conjugation of all the components.

### High Dispersibility

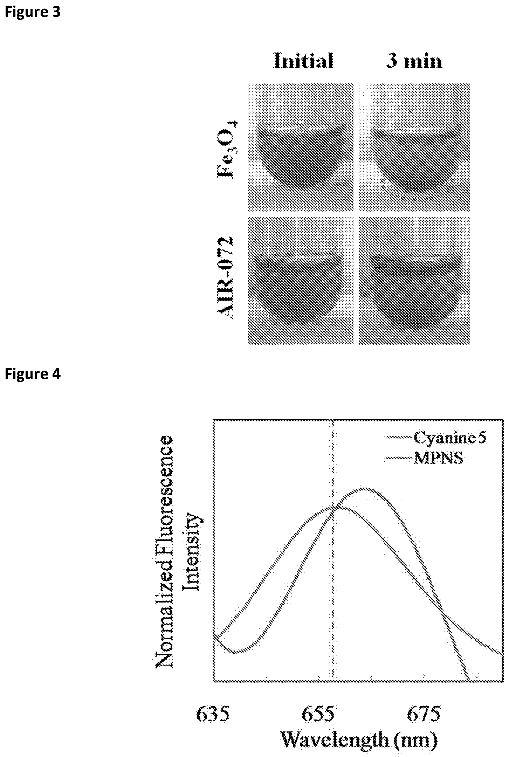

AIR-72 showed excellent dispersibility as compared to Fe₃O₄ nanoparticles. AIR-072 suspension showed uniform light brown color due to dispersed AIR-072 even after 3 min confirming its higher dispersion ability (**Figure 3**). However, in case of Fe₃O₄ nanoparticles most of the particles settled down after 3 min. The higher dispersibility of AIR-072 resulted from the presence of hydrophilic PAMAM G4 dendrimers and PNIPAM.

### Optical properties of MPNS

The conjugation of Cy5 into AIR-007 was confirmed by fluorescence measurements. Comparison of fluorescence spectrum (λex= 600 nm) of MPNS with those of free Cy5 is given in **Figure 4****.** The MPNS displayed the typical emission peak of Cy5 as shown in Figure 4. The fluorescence maxima of Cy5 showed a shift to the red upon conjugation with AIR-007 due to changes in conformation. This further confirms conjugation of Cy5 with AIR-007. The amount of Cy5 conjugated to MPNS was evaluated using UV-visible spectrophotmetry. About 60 µg of Cy5 was found to be conjugated per g of AIR-007.

### Tf conjugation to MPNS

Tf attachment on MPNS was quantified by Bradford procedure. The calibration curve was plotted by using BSA protein standard (50 µg/mL) in milliQ water. For estimating the amount of Tf conjugation, solution before and after Tf conjugation reaction for AIR-056 was taken in 96 well plate for analysis. 300 µL of 5X diluted Bio-rad protein assay reagent was added to each well and incubated for 5 minutes. The absorbance was measured at 570 nm on micro-plate reader. The amount of Tf conjugated was found to be 74.7 mg per gram of MPNS.

### Tf conjugated MPNS-Nanosystem mediated cell capturing

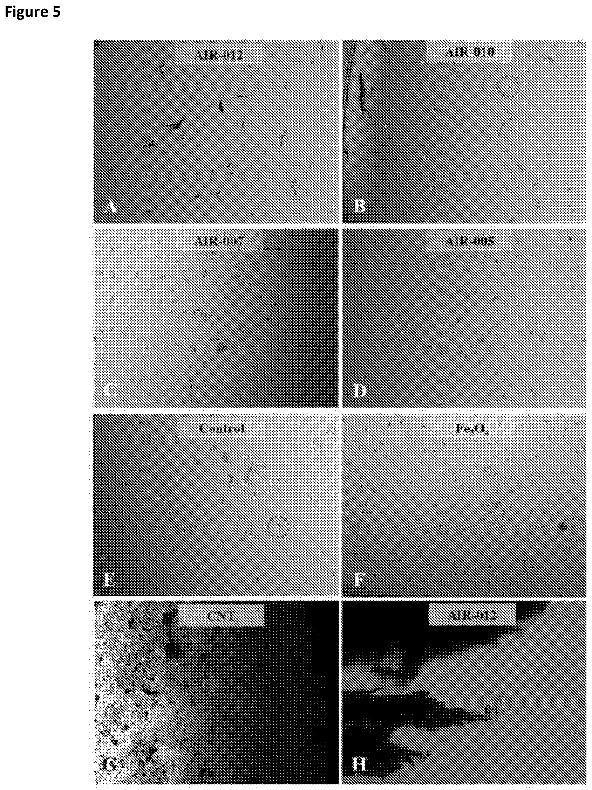

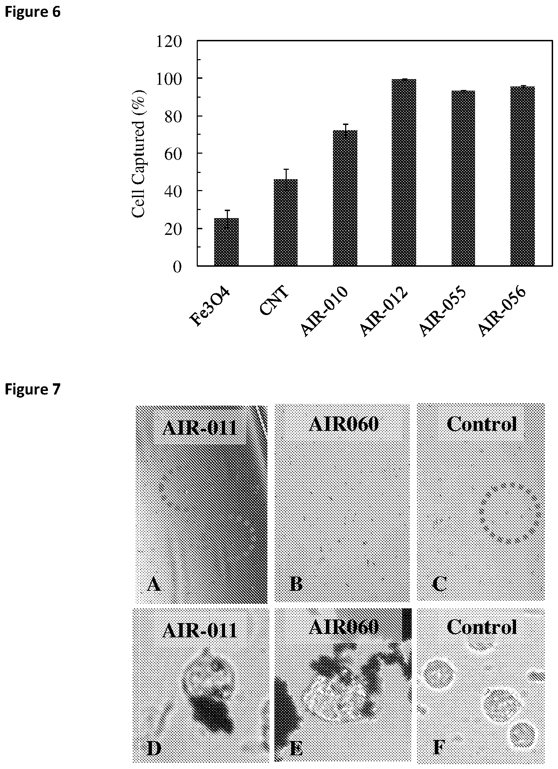

MPNS nanosystems - AIR-012, AIR-010 (with and without Tf), AIR-055, AIR-056 (with and without Tf) were evaluated for rapid capture of cancer cells by incubating with TfR⁺ colorectal carcinoma cell line HCT116 for 3 min. Furthermore, the components used for synthesizing MPNS nanosystems were also studied to assess non specific cell capture. It was observed that cell capturing ability of AIR-012 with Tf was higher than all other conjugates and components (**Figure 5**). The cell capture efficacy of MPNS was ∼ 100%. The cancer cell capturing ability was found AIR-012 > AIR-056 > AIR-055 > AIR-005 >> CNT > Fe₃O₄ (**Figure 6**).

### EpCam conjugated MPNS-Nanosystem mediated cell capturing

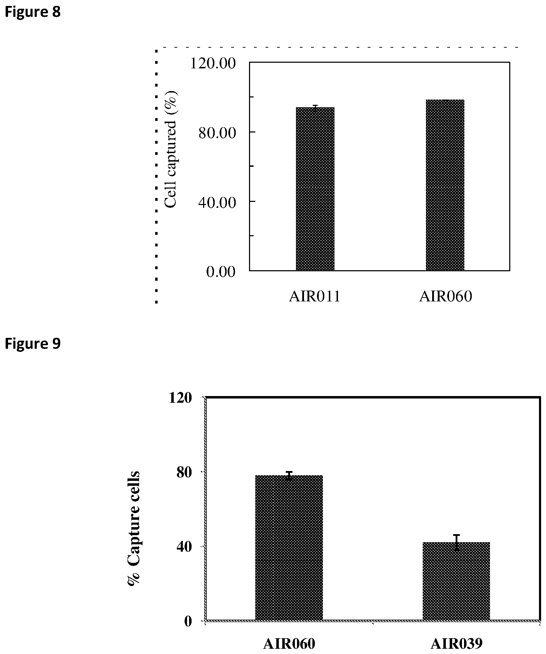

Cancer cell capture efficiency of MPNS with EpCam antibody was evaluated. Hence, MPNS nanosystems AIR-060 and AIR-011 (with and without EpCam) were evaluated by incubating with HCT116 cells for 3 min. We observed that cell capturing ability of AIR-060 with EpCam was higher than conjugate without EpCam (**Figure 7**). The cell capture efficacy of MPNS was ∼ 99% (**Figure 8**).

### EpCam conjugated MPNS-Nanosystem mediated capture efficiency from spiked CTC suspension

Cancer cell capture efficiency when mixed with hPBMCs of MPNS with EpCam antibody was evaluated. CTC samples were prepared by spiking hPBMCs with dual fluorescent probe labeled HCT116 cellsHCT116 cells at specific ratio (1:1000). Hence, MPNS nanosystems AIR-060 and AIR-039 (with and without EpCam) were evaluated by incubating artificial CTC suspension for 3 min. It was observed that cell capturing ability of AIR-060 with EpCam was higher than conjugate without EpCam. The cancer cell capture efficacy of MPNS with EpCam was ∼ 80% (**Figure 9**).

### EpCam conjugated MPNS-Nanosystem mediated cancer cell capturing

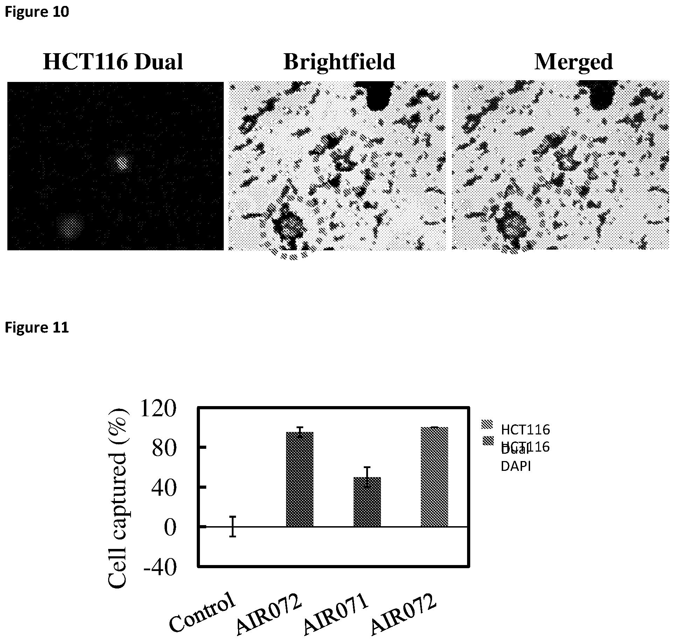

MPNS nanosystems AIR-072 and AIR-071 (with and without EpCam) were evaluated by incubating with a very low number of HCT116 cells (10 cells) for 3 min. It was observed that AIR-072 with EpCam had excellent capability in targeting and isolating HCT116 cells (**Figure 10**).

### EpCam conjugated MPNS-Nanosystem mediated capture efficiency from artificial CTC suspension of clinically relevant concentration

Cancer cell capture efficiency of MPNS in CTC samples at the clinically relevant concentrations (approximately one CTC per 10⁵ blood cells) was evaluated. CTC samples were prepared by spiking hPBMCs with GFP-labelled HCT116 cells at specific ratio (1:10⁵). MPNS nanosystems AIR-072 and AIR-071 (with and without EpCam) were evaluated by incubating for 3 min in CTC suspension. It was observed that cell capturing ability of AIR-072 with EpCam was higher than conjugate without EpCam. The cell capture efficacy of MPNS was ∼ 95 for dual fluorescent probe labeled HCT116 cells and 100% for DAPI stained HCT116 cells (**Figure 11**).

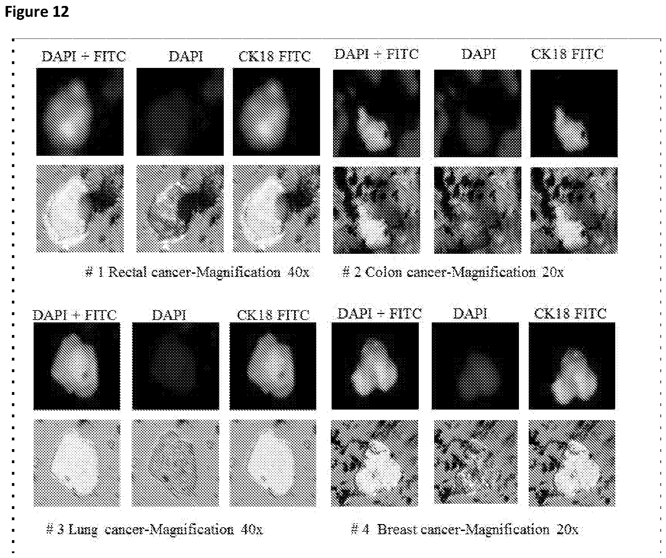

### CTC capture using cancer subjects (Table 1 and Figure 12)

AIR MPNS-EpCAM and graphene-EpCAM nanosystem were developed to isolate CTCs from cancer patient's whole blood samples. Blood samples from clinical cancer subjects were procured and RBCs were eliminated by treatment with RBC lysis buffer. Remaining sample was mixed with MPNS EpCAM or Graphene EpCAMnanosystem and were isolated with magnetic capturing. Further captured and uncaptured cells were fixed with formaldehyde and stained with Cytokeratin (CK)-18-FITC and CD45-PE to specifically detect cancer cells and blood cells (leucocytes) respectively (Figure 12).

Table 1. indicates the number of CTCs captured in rectal, colon, lung and breast cancer subjects.

**Table 1. CTC detected from cancer patient blood sample using AIR protocol**

| Type of Cancer | Clinical Status | No. of CTC detected | AIR CTC Remark |
|---|---|---|---|
| Rectal Cancer | Locally advanced non metastasis | 8/1.5ml blood | Metastasis+ |
| Colon Cancer | Locally advanced non metastasis | 8/1.5ml blood | Metastasis+ |
| Lung Cancer | Metastatic | 46/1.5ml blood | Metastasis+++ |
| Breast Cancer | Metastatic | 66/1.5ml blood | Metastasis+++ |

## Claims

1. A biofunctional multicomponent nanosystem comprising:
(i) a CTC specific or non-specific antibody targeting cancer cells, preferably an anti-transferrin (Tf) or anti-EpCam antibody, or a biomolecule targeting cancer cells;
(ii) iron oxide (Fe₃O₄) nanoparticles;
(iii) cyanine 5 NHS (Cy5) near infrared probe;
(v) carbon allotropes for interaction with cancer cells;
(vi) poly(N-isopropylacrylamide) (PNIPAM);
(vii) fourth generation (G4) dendrimers or polymers selected from the group consisting of polyglycerols, polyamines and hyperbranched polymers; and
(viii) glutathione (GSH) for specific targeting, isolation and imaging of circulating cancer cells.

2. The biofunctional multicomponent nanosystem of claim 1, wherein the said carbon allotrope is selected from the group consisting of single or multiwalled carbon nanotubes, graphene and nanohorns.

3. The biofunctional multicomponent nanysystem of claim 1, wherein the said fourth generation (G4) dendrimers or polymers are coupled to COOH, NH₂, OH or other reactive groups.

4. A process of synthesizing a biofunctional multicomponent nanosystem comprising the steps of:
a) synthesizing Fe₃O₄ magnetic nanoparticles by co-precipitating Fe²⁺ and Fe³⁺ ions by ammonia solution and treating under hydrothermal conditions;
b) anchoring of glutathione with Fe₃O_{4;}
c) synthesis of Fe₃O₄-GSH-PAMAM G4 dendrimer conjugate;
d) synthesis of Fe₃O₄-GSH-PAMAM G4-CNTor graphene conjugate;
e) synthesis of Fe₃O₄-GSH-PAMAM G4-CNTor graphene-Cy5-PNIPAM conjugate;
f) synthesis of Fe₃O₄-GSH-PAMAM G4-CNTor graphene-Cy5-PNIPAM-Tf conjugate; and
g) synthesis of Fe₃O₄-GSH-PAMAM G4-CNT or graphene-Cy5-PNIPAM-anti EpCam conjugate.

5. Use of the biofunctional multicomponent nanosystem of claims 1 to 3 or the product of the process according to claim 4 in the in vitro/ex vivo diagnosis of cancer.

6. A kit comprising a biofunctional multicomponent nanosytem comprising a CTC specific or non-specific antibody targeting cancer cells, preferably an anti-transferrin (Tf) or anti-EpCam antibody, iron oxide (Fe₃O₄) nanoparticles, cyanine 5 NHS (Cy5) near infra red probe, carbon allotropes, poly(N-isopropylacrylamide) (PNIPAM), fourth generation (G4) dendrimers or polymers like polyglycerols/polyimines and glutathione (GSH).

## Patentansprüche

1. Ein biofunktionelles Multikomponentennanosystem umfassend:
(i) einen auf Krebszellen gerichteten CTC-spezifischen oder unspezifischen Antikörper, vorzugsweise ein Anti-Transferrin (Tf) oder Anti-EpCam-Antikörper, oder ein auf Krebszellen gerichtetes Biomolekül;
(ii) Eisenoxid (Fe₃O₄)-Nanopartikel;
(iii) eine Cyanin 5 NHS (Cy5)-Nahinfrarotsonde;
(v) Kohlenstoffallotrope zur Wechselwirkung mit Krebszellen;
(vi) Poly(N-isopropylacrylamid) (PNIPAM);
(vii) Dendrimere der vierten Generation (G4) oder Polymers ausgewählt aus der Gruppe bestehend aus Polyglyzerolen, Polyaminen und hyperverzweigten Polymeren; und
(viii) Glutathion (GSH) zur spezifischen Ausrichtung auf, Isolierung und Bildgebung von zirkulierenden Krebszellen.

2. Das biofunktionelle Multikomponentennanosystem von Anspruch 1, wobei das genannte Kohlenstoffallotrop aus der Gruppe ausgewählt ist, bestehend aus einzel- oder mehrwandigen Kohlenstoffnanoröhrchen, Graphen und Nanohörnern.

3. Das biofunktionelle Multikomponentennanosystem von Anspruch 1, wobei die Dendrimere der vierten Generation (G4) oder Polymere an COOH, NH₂, OH oder andere reaktive Gruppen gebunden sind.

4. Ein Verfahren zur Synthese eines biofunktionellen Multikomponentennanosystems umfassend die folgende Schritte:
a) Synthetisieren magnetischer Fe₃O₄ Nanopartikel durch Ko-Präzipitation von Fe²⁺- und Fe³⁺-Ionen mittels Ammoniaklösung und Behandeln unter hydrothermalen Bedingungen;
b) Verankern von Glutathion mit Fe₃O_{4;}
c) Synthetisieren von Fe₃O₄-GSH-PAMAM G4-Dendrimer-Konjugat;
d) Synthetisieren von Fe₃O₄-GSH-PAMAM G4-CNT- oder Graphen- Konjugat;
e) Synthetisieren von Fe₃O₄-GSH-PAMAM G4-CNT- oder Graphen-Cy5-PNIPAM- Konjugat;
f) Synthetisieren von Fe₃O₄-GSH-PAMAM G4-CNT- oder Graphen-Cy5-PNIPAM-Tf- Konjugat; und
g) Synthetisieren von Fe₃O₄-GSH-PAMAM G4-CNT- oder Graphen-Cy5-PNIPAM-anti EpCam Konjugat.

5. Verwendung eines biofunktionellen Multikomponentennanosystems der Ansprüche 1 bis 3 oder des Produkts des Verfahrens gemäss Anspruch 4 in *der in vitro*/*ex vivo*-Diagnose von Krebs.

6. Ein Kit umfassend ein biofunktionelles Multikomponentennanosystem umfassend einen auf Krebszellen gerichteten CTC-spezifischen oder unspezifischen Antikörper, vorzugsweise einen Anti-Transferrin (Tf) oder Anti-EpCam-Antikörper, Eisenoxid (Fe₃O₄)-Nanopartikel, eine Cyanin 5 NHS (Cy5)-Nahinfrarotsonde, Kohlenstoffallotrope, Poly(N-isopropylacrylamid) (PNIPAM), Dendrimere der vierten Generation (G4) oder Polymers wie Polyglyzerole/Polyimine und Glutathion (GSH).

## Revendications

1. Nanosystème multicomposant biofonctionnel comprenant:
(i) un anticorps spécifique ou non spécifique de CTC ciblant des cellules cancéreuses, préférablement un anticorps anti-transferrine (Tf) ou anti-EpCam, ou une biomolécule ciblant des cellules cancéreuses;
(ii) des nanoparticules d'oxyde de fer (Fe₃O₄);
(iii) une sonde dans les proches infrarouges cyanine 5 NHS (Cy5);
(v) des allotropes carbonés pour une interaction avec les cellules cancéreuses;
(vi) du poly(N-isopropylacrylamide) (PNIPAM);
(vii) des dendrimères ou des polymères de quatrième génération (G4) sélectionnés dans le groupe constitué des polyglycérols, des polyamines et des polymères hyper-ramifiés; et
(viii) du glutathion (GSH) pour le ciblage, l'isolement et l'imagerie spécifiques de cellules cancéreuses circulantes.

2. Nanosystème multicomposant biofonctionnel selon la revendication 1, ledit allotrope carboné étant sélectionné dans le groupe constitué des nanotubes de carbone à paroi unique ou multiple, du graphène et des nanocornets.

3. Nanosystème multicomposant biofonctionnel selon la revendication 1, dans lequel lesdits dendrimères ou polymères de quatrième génération (G4) sont couplés à COOH, NH₂, OH ou à d'autres groupes réactifs.

4. Procédé de synthèse d'un nanosystème multicomposant biofonctionnel comprenant les étapes de:
a) synthèse de nanoparticules magnétiques de Fe₃O₄ par co-précipitation d'ions Fe²⁺ et Fe³⁺ par une solution d'ammoniaque et traitement sous des conditions d'hydrothermie;
b) ancrage de la glutathione avec du Fe₃O_{4;}
c) synthèse de conjugué de dendrimère Fe₃O₄-GSH-PAMAM G4;
d) synthèse de conjugué de Fe₃O₄-GSH-PAMAM G4-CNT ou de graphène;
e) synthèse de conjugué de Fe₃O₄-GSH-PAMAM G4-CNT ou de graphène-Cy5-PNIP AM;
f) synthèse de conjugué de Fe₃O₄-GSH-PAMAM G4-CNT ou de graphène-Cy5-PNIPAM-Tf; et
g) synthèse de conjugué de Fe₃O₄-GSH-PAMAM G4-CNT ou de graphène-Cy5-PNIPAM-anti-EpCam.

5. Utilisation du nanosystème multicomposant biofonctionnel selon les revendications 1 à 3 ou du produit du procédé selon la revendication 4 dans le diagnostic *in vitro*/*ex vivo* du cancer.

6. Kit comprenant un nanosystème multicomposant biofonctionnel comprenant un anticorps spécifique ou non spécifique de CTC ciblant des cellules cancéreuses, préférablement un anticorps anti-transferrine (Tf) ou anti-EpCam, des nanoparticules d'oxyde de fer (Fe₃O₄), une sonde dans les proches infrarouges cyanine 5 NHS (Cy5), des allotropes carbonés, du poly(*N-*isopropylacrylamide) (PNIPAM), des dendrimères de quatrième génération (G4) ou des polymères tels que des polyglycérols/polyimines et du glutathion (GSH).
